Europäisches Patentamt

European Patent Office

Office européen des brevets

(19)

(11) Numéro de publication: **0 234 065**
**B1**

(12) **FASCICULE DE BREVET EUROPEEN**

(45) Date de publication du fascicule du brevet:
08.11.89

(51) Int. Cl.⁴: **C13K 3/00**

(21) Numéro de dépôt: **86202397.5**

(22) Date de dépôt: **30.12.86**

(54) Procédé de traitement de jus sucré aqueux en vue de séparer et sélectionner les saccharides à fonction cétonique.

(30) Priorité: **17.01.86 FR 8601123**

(43) Date de publication de la demande:
**02.09.87 Bulletin 87/36**

(45) Mention de la délivrance du brevet:
**08.11.89 Bulletin 89/45**

(84) Etats contractants désignés:
**AT BE CH DE ES GB GR IT LI LU NL SE**

(56) Documents cités:
**FR-A- 1 511 419**
**FR-A- 2 099 297**

**CARBOHYDRATE RESEARCH,**
**vol. 107, 1982, pages 282-295, Elsevier Scientific**
**Publishing Co., Amsterdam, NL; F.J. LOPEZ et al.:**
**"Reactions of ketoses with ethyl acetoacetate"**
**BERICHTE DER DEUTSCHEN CHEMISCHEN**
**GESELLSCHAFT, vol. 72, no. 11, partie**
**B, 8 novembre 1939, pages 1993-1999, A. MÜLLER et al.:**
**"Die oxydative Spaltung der Polyoxy-Seitenketten in**
**den Zucker-Kondensationsprodukten des**
**Acetessigesters und des omicrom-Phenylendiamins"**
**000**
**CHEMICAL ABSTRACTS,**
**vol. 67, 1967, page 10284, abrégé no. 108947m,**

(73) Titulaire: **INSTITUT NATIONAL POLYTECHNIQUE DE TOULOUSE (I.N.P.T.), Place des Hauts-Murats B.P. 354, F-31006 Toulouse Cédex(FR)**

(72) Inventeur: **Jo, Louis Félix, 30 chemin des Maraîchers Bât.A-App. 12, F-31400 Toulouse(FR)**
Inventeur: **Borredon, Marie Elizabeth, 11 rue du Colonel Driant, F-31400 Toulouse(FR)**
Inventeur: **Delmas, Michel, 30 Résidence des Amazones, F-31320 Auzeville Tolosane(FR)**
Inventeur: **Gaset, Antoine, 75 Allée de Brienne, F-31000 Toulouse(FR)**

(74) Mandataire: **Barre, Philippe, Cabinet Barre-Gatti-Laforgue 95 rue des Amidonniers, F-31069 Toulouse Cédex(FR)**

(56) Documents cités: (suite)
**Columbus, Ohio, US; JP-B-8617/67 (FUJISAWA PHARMACEUTICAL CO., LTD) 13-07-1963**

ACTORUM AG

## Description

L'invention concerne un procédé de traitement de jus sucré aqueux contenant un mélange d'au moins un saccharide à fonction aldéhyde et d'au moins un saccharide à fonction cétonique. Elle vise à séparer et sélectionner les saccharides à fonction cétonique et à éliminer les saccharides à fonction aldéhyde sous la forme de sous-produits utilisables. Elle s'applique en particulier aux mélanges aqueux de fructose et de glucose en vue d'obtenir des sirops de fructose débarrassés du glucose.

On sait que, dans les industries du sucre et de l'amidon ou dans les industries de la conserverie, les jus de pressage des plantes sucrières ou effluents issus des diverses transformations des produits de base conduisent directement ou indirectement à des mélanges de saccharides aldéhydiques et saccharides cétoniques, essentiellement glucose et fructose.

Ces mélanges, lorsqu'ils sont suffisamment concentrés, sont utilisés, soit dans le domaine agro-alimentaire, soit, depuis peu, dans certains secteurs industriels (chimie fine).

Dans ces mélanges, le glucose constitue une charge gênante, aussi bien sur le plan industriel, car il conditionne des réactions parasites dans les processus ultérieurs, que sur le plan alimentaire, car il augmente considérablement l'apport calorique des sirops de fructose fabriqués à partir des jus sucrés.

Les deux processus essentiels de séparation du glucose des sirops de fructose utilisent, l'un des techniques chromatographiques, l'autre des techniques de cristallisation fractionnées. Le défaut majeur de ces procédés réside dans leur coût de mise en oeuvre, très élevé ; de plus, les techniques de cristallisation fractionnées ne s'adressent qu'à des sirops très riches en fructose qu'il s'agit de purifier.

La présente invention se propose de fournir un nouveau procédé de séparation des saccharides à fonction aldéhyde et des saccharides à fonction cétonique.

Un objectif essentiel de l'invention est de fournir un procédé de coût modéré, applicable aussi bien aux jus concentrés qu'à des jus plus dilués (effluents de sucrerie, amidonnerie, conserverie...), pour conduire à des solutions de saccharides cétoniques de qualité alimentaire.

Un autre objectif est de permettre une valorisation des saccharides aldéhydiques à éliminer, en les transformant en un produit utilisable dans différents secteurs industriels (notamment comme produit de base pour la fabrication de matériaux polymères).

Un autre objectif est d'obtenir des sirops de saccharides cétoniques, notamment de fructose, de haute pureté.

A cet effet, le procédé conforme à l'invention pour le traitement de jus sucré aqueux contenant un mélange d'au moins un saccharide à fonction aldéhyde et d'au moins un saccharide à fonction cétonique, en vue de séparer et sélectionner les saccharides à fonction cétonique, consiste, en combinaison :

(a) à confectionner un milieu hydro-organique réactif en mélangeant le jus sucré avec, d'une part, un réactif constitué par au moins un composé cétonique à méthylène actif, d'autre part, un réactif catalyseur à base de calcium (qualifié de catalyseur dans la suite),

(b) à agiter ledit milieu en vue de favoriser le contact des phases permettant la condensation du composé cétonique et des saccharides à fonction aldéhyde avec formation de polyol furannique.

(c) à laisser précipiter le polyol furannique dans le milieu réactionnel et à le séparer par voie physique du milieu liquide contenant les saccharides à fonction cétonique.

On a pu constater que les rendements de condensation sont très améliorés lorsque le milieu hydro-organique contient de l'éthanol ou du méthanol, qui seront de préférence additionnés au milieu lors de l'opération (a). Aucun produit de dégradation ne vient alors polluer le milieu après réaction.

Ainsi, les travaux des inventeurs ont mis en évidence une réaction de condensation qui, de façon inattendue, possède la propriété remarquable d'être totalement sélective vis-à-vis des saccharides aldéhydiques lorsqu'ils se trouvent mélangés avec des saccharides cétoniques : en milieu hydro-organique (préférentiellement eau/éthanol ou méthanol) et en présence d'un catalyseur à base de calcium, cette réaction fait subir une transformation stoechiométrique aux saccharides aldéhydiques et notamment au glucose, et préserve dans son intégralité les saccharides cétoniques et notamment le fructose. Les saccharides aldéhydiques sont transformés en polyols furanniques sans réaction secondaire : ce corps précipite spontanément dans le milieu réactionnel hydro-organique et peut ainsi être séparé, en particulier par simple filtration, pour conduire à une phase liquide contenant uniquement les saccharides à fonction cétonique.

Selon un mode de mise en oeuvre préféré, le milieu hydro-organique est confectionné en ajustant la quantité d'alcool par rapport à la quantité d'eau, de façon que la proportion volumique relative d'alcool dans l'eau soit approximativement comprise entre 80 et 95 %. De plus, le solvant utilisé est très avantageusement de l'éthanol. Ces conditions conduisent à un rendement très élevé de séparation (généralement supérieur à 95 %) et permettent d'obtenir une solution de saccharides cétoniques de qualité alimentaire.

Le composé cétonique à méthylène actif est de préférence mélangé légèrement en excès par rapport aux proportions stoechiométriques vis-à-vis des saccharides à fonction aldéhyde contenu dans le jus sucré, en vue d'obtenir une transformation totale de ces saccharides aldéhydiques en polyol furannique. De plus, le catalyseur à base de calcium, notamment chlorure de calcium, peut être mélangé dans une proportion molaire comprise entre 1 et 6 par rapport aux saccharides à fonction aldéhyde contenu dans le jus sucré.

Le réactif cétonique est notamment constitué par de l'acétylacétate d'éthyle ou de méthyle ou de l'acétylacétone, qui ont l'avantage de ne présenter aucun caractère toxique en cas de traces résiduelles.

Pour réduire la durée de la réaction, le milieu réactionnel est avantageusement chauffé à une température comprise entre 40° C et la température de reflux du milieu réactionnel, pendant une durée comprise entre 4 et 8 heures. En pratique, on pourra se situer au voisinage immédiat de la température de reflux du solvant, notamment 78° C dans le cas de l'éthanol (reflux de l'azéotrope éthanol/eau).

La précipitation du polyol s'effectue, de préférence à froid et peut être favorisée par un ajout d'eau dans le milieu après la réaction de condensation.

On conçoit l'intérêt du procédé de séparation conforme à l'invention, qui délivre des sirops de saccharides cétoniques totalement débarrassés des saccharides aldéhydiques ; ces sirops se prêtent à une utilisation industrielle ou agro-alimentaire sans réaction parasite générant des produits toxiques et sans apport calorique défavorable lié à la présence de glucose.

Il est à noter que des travaux en laboratoire ont déjà été menés sur la réaction de condensation du glucose pur (ou d'autres sucres aldéhydiques purs) en vue de synthétiser des polyols furanniques. On pourra se reporter aux publications suivantes qui évoquent cette réaction : F.J. LOPEZ APARICIO et al, Carbohydrate Research, vol. 107, 1982, pages 292-295 ; F. GARCIA-GONZALEZ, Adv. Carbohydr. Chim. 1956, 11, p 111 ; J.S. BALLESTEROS, D.J. McPHEE, D. HERNANDEZ, Bull. Soc. Chim. FR 1982, p 176 ; M. OKADA, Y. KASHIWABARA, T. IMAMURA, M. YAMADA, M. KAKEHI; Chemical Abstracts, 1967, 67, n ° 108947 M.

Toutefois, ces travaux visent simplement une réaction de condensation d'espèces pures en milieu synthétique et non une séparation d'une espèce dans un mélange, et ne décrivent ni ne suggèrent aucune propriété de sélectivité de cette réaction. Bien au contraire, ces publications, et notamment la première citée, montrent que dans les conditions de mise en oeuvre et compte-tenu des catalyseurs utilisés, la réaction de condensation affecte indistinctement aussi bien le glucose que le fructose. Ils ne fournissent donc aucun enseignement directement utilisable pour résoudre le problème de séparation visé par l'invention. En outre, ces travaux préconisent des conditions particulières de mise en oeuvre ou un type spécifique de catalyseurs (acides de Lewis, notamment $Zn Cl_2$) difficilement exploitables sur le plan industriel en raison des graves défauts qui en sont la conséquence : reproductibilité aléatoire, pollution métallique des phases aqueuses, difficultés de récupération des polyols furanniques qui cristallisent mal, rendement maximum ( de l'ordre de 60 %).

Les exemples qui suivent illustrent le procédé de séparation conforme à l'invention.

## EXEMPLE 1

On hydrolyse 100 g de saccharose (sucre de canne) par 46 g de résine cationique "Amberlit E 252" vendu par la Société "Rohm and Haas". Cette réaction a lieu dans 50 ml d'eau et 160 ml d'éthanol à 80° C.

Après avoir séparé la résine du milieu réactionnel et dosé la quantité de glucose contenue dans la solution, on mélange 93 ml de cette solution contenant 18 g de glucose, 7 ml d'éthanol, 55 g de chlorure de calcium et 26,5 ml d'acétylacétate d'éthyle.

On agite le milieu et on le chauffe pour le porter à 78° C (température de reflux) pendant 6 h 30.

Pendant la réaction, l'on observe une précipitation de cristaux blanchâtres ; en fin de réaction, 400 ml d'eau sont ajoutées et les cristaux sont filtrés.

La solution mère est laissée au repos ; puis une nouvelle filtration de cette solution est effectuée et permet à nouveau de récupérer des cristaux de furoate d'éthyle 4-(D-arabino-tétroxybutyle)2-méthyl 5.

Le filtrat résultant est soumis à deux étapes de purification :
- extraction de l'éthanol du milieu par distillation douce, et extraction de l'acétylacétate d'éthyle en excès par l'acétate d'éthyle en vue d'un recyclage,
- déminéralisation et décoloration simultanées par échangeurs d'ions ("Lewatit SP 102/MP 64" vendu par la Société "BAYER").

La solution obtenue est déshydratée et fournit un sirop de fructose pur d'une teneur supérieure à 95 % en poids de fructose, exempt d'acétylacétate d'éthyle, de sel et d'éthanol. Le fructose cristallise par addition d'éthanol au sirop obtenu.

## EXEMPLE 2

L'hydrolyse est réalisée selon l'exemple 1 mais sans ajout d'éthanol ; la concentration initiale de saccharose est de 32,4 g pour 100 ml d'eau. Après hydrolyse, on concentre le milieu jusqu'à l'obtention d'un sirop de sucre et on introduit pour 18 g de glucose, 30 g de chlorure de calcium, puis on verse à l'aide d'une ampoule à Brome 9 ml d'acétylacétone.

Ce milieu est porté à 80° C pendant 5 h et les cristaux furanniques produits sont filtrés puis purifiés donnant l'acétyl 4-(D-arabino-tétroxybutyle) 2-méthyl 5-furanne.

Le filtrat résultant est soumis à une déminéralisation donnant en fin d'opération du sirop de fructose d'une teneur en fructose de 60 % en poids. On constate toutefois une dégradation du glucose qui produit une certaine pollution du sirop, nécessitant une purification spécifique. On préfèrera donc utiliser un milieu organique alcoolique.

## EXEMPLE 3

Dans cet exemple, on traite des mélasses de cannes à sucre pour lesquelles la proportion de saccharides varie de 39 à 61 %.

On introduit 100 g de mélasse de canne dans un réacteur. De l'acide chlorhydrique est ensuite ajouté jusqu'au pH = 2. Le tout est porté à 85° C durant environ 1/2 heure; l'inversion est contrôlée par chromatographie liquide haute performance (H.P.L.C.). Le mélange est neutralisé par addition lente de chaux (pH 6-7).

Après inversion. le procédé de l'invention est mis en oeuvre : on introduit du chlorure de calcium (12 g pour 10 g de saccharides) et 23 g d'acétylacétate de méthyle pour 18 g de glucose, puis 150 ml d'éthanol.

Le mélange est porté à 78° C pendant 6 h 30 ; 200 ml d'eau sont additionnés en fin de réaction et les cristaux furanniques formés sont séparés de la solution mère pour donner le furoate de méthyl 4-(D-arabino tétroxybutyle)2-méthyl 5.

On obtient un sirop de fructose pur à une teneur de 90 % en fructose.

## EXEMPLE 4

Dans cet exemple, on traite des mélasses de betteraves dont la teneur totale en saccharide varie de 37 à 75 % en poids.

L'inversion est réalisée par de l'acide chlorhydrique (8 ml à 33 %) ajouté pour 100 g de mélasse de betterave.

On porte le mélange à 80° C ; le contrôle du taux de conversion du saccharose en glucose et fructose est effectué par "H.P.L.C."

Après neutralisation du milieu, le chlorure de calcium est introduit (12 g pour 10 g de saccharides), puis l'acétylacétate d'éthyle (26 g pour 18 g de glucose), enfin 150 ml d'éthanol sont ajoutés.

L'ensemble porté à 78° C est agité durant 6 h 30, puis de l'eau est introduite pour produire après plusieurs recristallisations du furoate d'éthyl 4-(D-arabino-tétroxybutyle)2-méthyl 5.

Après cristallisation et filtration, le filtrat précédent est purifié de façon analogue à l'exemple 1 pour produire du sirop de fructose pur de teneur en fructose égale à 93 %.

## EXEMPLE 5

A un mélange de saccharides à fonctions aldéhyde et cétonique contenant 18 g de glucose pour 20 ml d'eau, on introduit 80 ml d'éthanol, 55,5 g de chlorure de calcium et 31,64 g d'acétylacétate de butyle.

Le milieu réactionnel est porté à reflux de l'azéotrope eau-éthanol (78° C) durant 6 h 30 et conduit à la formation du furoate d'éthyle 4-(D-arabino-tétroxybutyle)2-propyl 5.

L'éthanol et la β dicétone sont extraits du filtrat ; ce dernier est ensuite débarrassé du catalyseur.

Le sirop de fructose obtenu possède une teneur pondérale en fructose de 90 %.

## EXEMPLE 6

Une fraction d'un sirop de sucres invertis de concentration en glucose égale à 900 g/l est mis en contact avec 80 ml d'éthanol, 55,5 g de chlorure de calcium et 38,4 g de benzoylacétate d'éthyle.

L'ensemble est porté à 78° C durant 6 h 30 pour donner le furoate d'éthyle 3-(D-arabino-tétroxybutyle)5-phényl 2.

Le filtrat recueilli est purifié pour produire un sirop de fructose d'une teneur pondérale égale à 70 % en poids.

## Revendications

1/ - Procédé de traitement de jus sucré aqueux contenant un mélange d'au moins un saccharide à fonction aldéhyde et d'au moins un saccharide à fonction cétonique, en vue de séparer et sélectionner les saccharides à fonction cétonique, caractérisé en ce qu'il consiste en combinaison :

(a) à confectionner un milieu hydro-organique réactif en mélangeant le jus sucré avec, d'une part, un réactif constitué par au moins un composé cétonique à méthylène actif, d'autre part, un catalyseur à base de calcium,

(b) à agiter ledit milieu en vue de favoriser le contact des phases permettant la condensation du composé cétonique et des saccharides à fonction aldéhyde avec formation de polyol furannique,

(c) à laisser précipiter le polyol furannique dans le milieu réactionnel et à le séparer par voie physique du milieu liquide contenant les saccharides à fonction cétonique.

2/ - Procédé selon la revendication 1, caractérisé en ce que (a) l'on confectionne le milieu hydro-organique en ajoutant de l'éthanol ou du méthanol.

3/ - Procédé selon la revendication 2, caractérisé en ce que (a) le milieu hydro-organique est confectionné en ajustant une quantité d'alcool par rapport à la quantité d'eau de façon que la proportion pondérale relative d'alcool dans l'eau soit approximativement comprise entre 80 et 95 %.

4/ - Procédé selon l'une des revendications 1, 2 ou 3, caractérisé en ce que le composé cétonique à méthylène actif est mélangé légèrement en excès par rapport aux proportions stoechiométriques vis-à-vis des saccharides à fonction aldéhyde contenus dans le jus sucré.

5/ - Procédé selon l'une des revendications 1, 2, 3 ou 4, caractérisé en ce que le catalyseur à base de calcium est mélangé dans une proportion molaire comprise entre 1 et 6 par rapport aux saccharides à fonction aldéhyde contenus dans le jus sucré.

6/ - Procédé selon l'une des revendications 2 ou 3, caractérisé en ce que l'on utilise, comme alcool, de l'éthanol.

7/ - Procédé selon l'une des revendications 1, 2, 3, 4, 5 ou 6, dans lequel le réactif est constitué par de l'acétylacétonate d'éthyle ou de méthyle.

8/ - Procédé selon l'une des revendications 1, 2, 3, 4, 5 ou 6, dans lequel le réactif est constitué par de l'acétylacétone.

9/ - Procédé selon l'une des revendications 1, 2, 3, 4, 5, 6, 7 ou 8, dans lequel le catalyseur est du chlorure de calcium.

10/ - Procédé selon l'une des revendications précédentes, dans lequel (b) le milieu réactionnel est chauffé à une température comprise entre 40° C et la température de reflux dudit milieu, pendant une durée comprise entre 4 et 8 heures.

11/ - Procédé selon l'une des revendications précédentes, dans lequel la précipitation (c) est réalisée à froid.

12/ - Procédé selon l'une des revendications précédentes, caractérisé en ce que, après réaction (b), l'on ajoute de l'eau dans le milieu en vue de favoriser la précipitation.

13/ - Procédé selon l'une des revendications précédentes, dans lequel la séparation de polyol furannique est réalisée par filtration.

14/ - Procédé selon l'une des revendications précédentes, appliqué à un mélange aqueux de fructose et de glucose en vue d'obtenir un sirop de fructose débarrassé du glucose.

## Claims

1. Process for the treatment of an aqueous sugar solution containing a mixture of at least one saccharide having an aldehyde function and at least one saccharide having a ketonic function, in order to isolate and separate the saccharides having a ketonic function, characterised in that it consists of the combination:
   a) preparing a hydro-organic reagent medium by mixing the sugar solution with, on the one hand, a reagent comprising at least one ketonic compound with an active methylene group, and, on the other hand, a catalyst based on calcium,
   b) stirring said medium in order to favour the contact of phases which allow the condensation of the ketonic compound and of the saccharides having an aldehyde function with formation of furane polyol,
   c) allowing the furane polyol to precipitate in the reactive medium, and separating it by a physical method from the liquid medium containing the saccharides having a ketonic function.

2. Process according to claim 1, characterised in that the hydro-organic medium (a) is prepared by adding ethanol or methanol.

3. Process according to claim 2, characterised in that in (a) the hydro-organic medium is created by adjusting an amount of alcohol with regard to the amount of water in such a way, that the relative proportion by weight of the alcohol in the water is between approximately 80 and 95%.

4. Process according to one of claims 1, 2 or 3, characterised in that the ketonic compound with an active methylene group is used slightly in excess with regard to the stoichiometrical proportions of the saccharides having an aldehyde function contained in the sugar solution.

5. Process according to one of claims 1, 2, 3, or 4, characterised in that the calcium-based catalyst is mixed in a molar proportion between 1 and 6 with regard to the saccharides having an aldehyde function contained in the sugar solution.

6. Process according to one of claims 2 or 3, characterised in that ethanol is used as an alcohol.

7. Process according to one of claims 1, 2, 3, 4, 5, or 6, characterised in that the reagent is composed of ethyl- or methyl-acetylacetonate.

8. Process according to one of claims 1, 2, 3, 4, 5 or 6, wherein the reagent is composed of acetylacetone.

9. Process according to one of claims 1, 2, 3, 4, 5, 6, 7 or 8, wherein the catalyst is calciumchloride.

10. Process according to one of the preceding claims, characterised in that the reacitve medium in (b) is heated to a temperature between 40°C and the temperature of reflux of the said medium for a period between 4 and 8 hours.

11. Process according to one of the preceding claims, characterised in that the precipitation (c) is produced in cold conditions.

12. Process according to one of the preceding claims, characterised in that, after reaction (b), water is added to the medium to speed up the precipitation.

13. Process according to one of the preceding claims, characterised in that the separation of the furane polyol is realised by filtration.

14. Process according to one of the preceding claims, applied to an aqueous mixture of fructose and glucose with a view to obtaining a fructose syrup free from glucose.

## Patentansprüche

1. Verfahren zur Behandlung einer wässrigen Zuckerlösung, die eine Mischung wenigstens eines Saccharides mit einer Aldehydgruppe und wenigstens eines Saccharides mit einer Ketongruppe enthält, zu dem Zweck, Saccharide mit einer Ketongruppe zu isolieren und zu trennen, dadurch gekennzeichnet, dass es aus der folgenden Kombination besteht:
   a) Herstellung eines hydro-organisch reagenten Mediums durch Mischen der Zuckerlösung einerseits mit einer Reagens, die aus wenigstens einer ketonischen Verbindung mit einer aktiven Methylengruppe besteht, und andererseits mit einem Katalysator mit Calcium als Grundlage,
   b) Umrührung des besagten Mediums, um den Kontakt der Phasen zu fördern, die die Kondensation der ketonartigen Verbindung und der Saccharide mit einer Aldehydgruppe gestatten, um ein Furanpolyol zu formen,
   (c) Gewährung der Ausfällung des Furanpolyols in dem reaktiven Medium, und ihrer Trennung von dem flüssigen Medium, welches die Saccharide mit einer Ketongruppe enthält, durch eine physikalische Methode.

2. Verfahren nach Anspruch 1, dadurch gekennzeichnet, dass das hydro-organische Medium (a) dadurch zubereitet wird, dass man Äthanol oder Methanol hinzusetzt.

3. Verfahren nach Anspruch 2, dadurch gekennzeichnet, dass das hydro-organische Medium in (a) dadurch hergestellt wird, indem eine Alkoholmenge in bezug auf die Wassermenge so justiert wird, dass das relative Gewichtsverhältnis des Alkohols im Wasser ungefähr 80 bis 95% beträgt.

4. Verfahren nach einem der Ansprüche 1, 2, oder 3, dadurch gekennzeichnet, dass die ketonische Verbindung mit aktiver Methylengruppe leicht im Übermaß in bezug auf die stöchiometrischen Verhältnisse der Saccharide mit einer Aldehydgruppe in der Zuckerlösung enthalten ist.

5. Verfahren nach einem der Ansprüche 1, 2, 3 oder 4, dadurch gekennzeichnet, dass der auf Calcium beruhende Katalysator in einem molaren Verhältnis von 1 bis 6 in bezug auf die Saccharide mit einer Aldehydgruppe, die in der Zuckerlösung enthalten sind, gemischt ist.

6. Verfahren nach einem der Ansprüche 2 oder 3, dadurch gekennzeichnet, dass Äthanol als Alkohol verwandt wird.

7. Verfahren nach einem der Ansprüche 1, 2, 3, 4, 5 oder 6, dadurch gekennzeichnet, dass das Reagens aus Äthyl- oder Methyl-acetylacetonat besteht.

8. Verfahren nach einem der Ansprüche 1, 2, 3, 4, 5 oder 6, dadurch gekennzeichnet, dass das Reagens aus Acetylaceton besteht.

9. Verfahren nach einem der Ansprüche 1, 2, 3, 4, 5, 6, 7 oder 8, dadurch gekennzeichnet, dass der Katalyst aus Calciumchlorid besteht.

10. Verfahren nach einer der vorhergehenden Ansprüche, dadurch gekennzeichnet, dass das reaktive Medium in (b) auf eine Temperatur zwischen 40°C und der Rückflusstemperatur des besagten Mediums in einer Dauer zwischen 4 und 8 Stunden erhitzt wird.

11. Verfahren nach einem der vorhergehenden Ansprüche, dadurch gekennzeichnet, dass die Ausfällung (c) in kalten Bedingungen hergestellt wird.

12. Verfahren nach einem der vorhergehenden Ansprüche, dadurch gekennzeichnet, dass nach Reaktion (b) Wasser zum Medium hinzugefügt wird, um die Ausfällung zu beschleunigen.

13. Verfahren nach einem der vorhergehenden Ansprüche, dadurch gekennzeichnet, dass die Trennung des Furanpolyols durch Filtration realisiert wird.

14. Verfahren nach einer der vorhergehenden Ansprüche, angewandt auf eine wässrige Mischung von Fructose und Glucose, um Fructosesirup frei von Glucose zu erhalten.